# EUROPEAN PATENT APPLICATION

(11) **EP 4 331 657 A1**
(43) Date of publication of application: **06.03.2024**
(21) Application number: 21938374.2
(22) Date of filing: 29.04.2021
(51) Int. Cl.: A61M 29/00, A61M 15/08, A62B 9/00

(54) **SLOW RELEASE APPARATUS FOR SLOWLY RELEASING COMPOUND IN NASAL PASSAGE**

(71) Applicant: Wang, Lei, Beijing 100085 (CN)
(72) Inventor: Wang, Lei, Beijing 100085 (CN)
(74) Representative: Bryn Aarflot AS
(86) International application number: PCT/CN2021/090988
(87) International publication number: WO 2022/226894

(57) **Abstract**

A slow release apparatus for slowly releasing a compound in a nasal passage. The apparatus comprises a connection mechanism, two expansion mechanisms, and two release mechanisms; two ends of the connection mechanism are respectively integrally or detachably connected to the two expansion mechanisms that are symmetrically arranged in nasal passages at two sides; and the two release mechanisms are symmetrically arranged, and each release mechanism is correspondingly provided in one of the expansion mechanisms. The slow release apparatus is convenient to carry, and capable of slowly releasing a compound in the nasal passage and synchronously expanding the nasal passage to ensure smooth breathing, while considering functionality, practicability and comfort.

## Description

### TECHNICAL FIELD

The present invention belongs to the technical field of medical devices, and relates to a slow release apparatus for slowly releasing a compound in a nasal passage.

### BACKGROUND

Nasal sprays are commonly used to treat allergic rhinitis and nasal passage obstruction. Active ingredients of such medicine stimulate hormone receptors in the nose, causing vasoconstriction to relieve nasal mucosa congestion and nasal obstruction. As a large dose of medicine is brought into direct contact with the nasal mucosa in short time, the number of the receptors rapidly decreases, such that the nasal mucosa becomes more congested and inflamed after a period of time, and blood vessels become brittle and prone to nosebleeds. Slow release technology can avoid an instantaneous impact of medicine on the nasal mucosa, thereby weakening or even avoiding the occurrence of the above-mentioned side effects. In addition, the use of essential oils to improve nausea, allergies, insomnia and other conditions has become widely known. Traditional aroma diffusers are mostly placed in the room, and are large in volume and inconvenient to carry, while portable nasal slow release apparatuses can help people use essential oils and other aromatic compounds anywhere, anytime. The prior art provides various portable nasal clips to assist people in slow release of compounds in nasal passages, but the existing nasal clips often can only release compounds of specific types or states, lack versatility, and have great limitations in release effect and ventilation.
1. A type of nasal clip is made of a material doped with a volatile aromatic compound. Such a nasal clip is simple in structure and easy to wear, but the aromatic compound directly contacts and irritates an inner wall of the nasal passage; and the aromatic compounds carried therein cannot be replaced. In addition, such a nasal clip does not have a function of expanding the nasal passage, and therefore impedes breathing to some extent.
2. A type of nasal clip is provided with an openable-closable cavity inside, in which a compound is placed, and the compound is released into the nasal passage through an opening in the cavity. However, due to providing the cavity, this type of nasal clip is relatively thick and big, and it does not have a function of expanding the nasal passage, and therefore impedes breathing to some extent. In addition, as an opening and closing device of the cavity is small, complicated, and low in reliability, it may cause a risk of being sucked in once it fails.
3. A type of nasal clip is provided with a round tube at a tail end, with a solid-state aromatic compound being placed in the round tube. The solid-state compound is fixed by a narrowed part in the middle of the round tube. However, this type of nasal clip cannot release a liquid compound. Moreover, since the solid-state aromatic compound is not hard, its fixation mode is not reliable enough, which may cause a risk of being sucked in. In addition, this type of nasal clip does not have a function of expanding the nasal passage, and therefore impedes breathing to some extent.
4. A type of nasal clip is provided with an expansion element for expanding the nasal passage, and a release element fixed to the expansion element to release a compound. The ventilation of this type of nasal clip is better than that of other nasal clips, but the contact area between its expansion element and an inner wall of the nasal passage is smaller, and the intensity of pressure on the inner wall of the nasal passage is greater, which is liable to cause discomfort and makes it difficult to expand the nasal passage in all directions. In addition, the expansion element is not provided with an elastic support spoke, and therefore is inadequate in strength and balance of expansion, and it is difficult to expand specific parts of the nasal passages in a targeted manner. Furthermore, its release element can only contain a small amount of compound of a specific state and type, and its opening and closing device is small, complicated, and not reliable enough.
5. A type of nasal clip has both filtering and releasing functions. It includes a pair of cylindrical containers connected together by a connecting band. Each cylindrical container is internally provided with a filter and an absorption washer capable of absorbing liquid-state medicine. The absorption washer can release the medicine into an inspiratory airflow. However, this type of nasal clip cannot release a solid-state compound; and inhaled gas passes through the filter and the absorption washer, leading to high respiratory resistance, so it is less versatile and is only suitable for specific situations.

### SUMMARY OF THE INVENTION

To solve the above-mentioned shortcomings of the prior art, the present invention proposes a slow release apparatus for slowly releasing a compound in a nasal passage, wherein the slow release apparatus includes a connection mechanism, two expansion mechanisms, and two release mechanisms; two ends of the connection mechanism are respectively integrally or detachably connected to the two expansion mechanisms that are symmetrically arranged in nasal passages at two sides; and the two release mechanisms are symmetrically arranged, and each release mechanism is correspondingly provided in one of the expansion mechanisms;
the connection mechanism includes at least one connecting beam, the connecting beam semi-surrounding a lower end of a nasal septum and including an intermediate portion located on a lower side of the nasal septum and two connecting arms symmetrically arranged on two sides of the nasal septum; the intermediate portion is located between the two connecting arms; the two connecting arms are respectively connected to the two expansion mechanisms symmetrically arranged in the two nasal passages;
the release mechanism includes at least one fixing tube and at least one release element;
the fixing tube is a tubular structure, which is arranged on the expansion mechanism; the release element is a structure wide at two ends and narrow in the middle, and when worn, the release element is fixed in the fixing tube, the fixing tube surrounds the thinner middle of the release element and is tightly engaged therewith, the two ends of the release element are located on outer sides of two ends of the fixing tube, and maximum widths of the two ends of the release element are greater than a maximum width of an inner cavity of the fixing tube; and the release element is capable of containing a certain amount of compound and allows the contained compound to be slowly released in the nasal passage, and when worn, at least part of the release element is located in the nasal passage.

As one of improvements of the above technical solution, the expansion mechanism includes an elastic expansion arm; the elastic expansion arm is a ring-like closed structure, which is connected to an end of one connecting arm; the fixing tube is arranged on an inner side primary surface of the elastic expansion arm, and the release element is sleeved in the fixing tube.

As one of improvements of the above technical solution, the two connecting arms of the connecting beam are symmetrically provided with widened portions, and the width of a surface on a side of each connecting arm facing towards the nasal septum increases at the widened portion; and the two widened portions are fit to two sides of the nasal septum and apply a clipping force to the nasal septum.

As one of improvements of the above technical solution, the expansion mechanism includes an elastic expansion arm and at least one elastic support spoke;
the elastic expansion arm is a ring-like closed structure, which is connected to an end of one connecting arm; the elastic support spoke is forked and includes a plurality of branches, each branch having one end connected to a trunk part of the elastic support spoke, and the other end fixed to the elastic expansion arm; and the fixing tube is arranged on the elastic support spoke, and the release element is sleeved in the fixing tube.

As one of improvements of the above technical solution, the elastic support spoke, the fixing tube and the release element are located above the elastic expansion arm, forming an inverted bowl structure.

As one of improvements of the above technical solution, the expansion mechanism includes an elastic expansion arm and at least one elastic support spoke;
the elastic expansion arm is a ring-like closed structure, which is connected to an end of one connecting arm; and the elastic support spoke is a band-like structure, two ends of which are connected to the elastic expansion arm.

As one of improvements of the above technical solution, the connection mechanism includes a first connecting beam and a second connecting beam arranged side by side longitudinally; and both the first connecting beam and the second connecting beam semi-surround the lower end of the nasal septum.

As one of improvements of the above technical solution, two second connecting arms of the second connecting beam are symmetrically provided with protrusions towards the nasal septum of a wearer; and the protrusions are fit to the two sides of the nasal septum and apply a clipping force to the nasal septum.

As one of improvements of the above technical solution, ends of two second connecting arms of the second connecting beam are provided with plugs, the plugs being inserted into jacks provided at roots of first elastic support spokes, each plug being a structure wide at two ends and narrow in the middle to achieve plug-in fixation.

As one of improvements of the above technical solution, the expansion mechanism includes an elastic expansion arm, a nasal passage engagement element and at least one elastic support spoke;
the elastic expansion arm is an elastically compressible open band-like structure, which is connected to an end of one connecting arm, and an outer side primary surface of the elastic expansion arm is configured to be partially or entirely fit to an inner wall of the nasal passage, so as to be capable of applying an expansion force from the elastic expansion arm and the elastic support spoke to a nasal wing and applying a clipping force from the connecting beam to the nasal septum;
the nasal passage engagement element is a sheet-like structure, which is connected to the elastic expansion arm and keeps the expansion mechanism as an open structure; when worn, the nasal passage engagement element is located on an inner side of the nasal wing, and an outer primary surface thereof is fit to an inner wall of the nasal wing and applies an expansion force from the elastic expansion arm and the elastic support spoke to the nasal wing; and
the elastic support spoke is an elastically compressible band-like or strip-like structure, two ends of which are connected to the elastic expansion arm or the nasal passage engagement element.

As one of improvements of the above technical solution, the elastic expansion arm is fused with the nasal passage engagement element into an integral whole.

As one of improvements of the above technical solution, a transverse cross-section of the elastic expansion arm perpendicular to a nasal passage axis is a V-shaped structure or C-shaped structure including a bent portion, a proximal arm and a distal arm;
the bent portion is connected to the proximal arm at one end and to the distal arm at the other end; and when worn, the proximal arm is close to the nasal septum, the distal arm is close to the nasal wing, and the bent portion is close to a nasal bridge or a nasal base.

As one of improvements of the above technical solution, an end of the elastic expansion arm, at a part fit to the inner wall of the nasal passage, is bent towards an inner side of the nasal passage; and an end of the nasal passage engagement element is bent towards the inner side of the nasal passage.

As one of improvements of the above technical solution, the elastic support spoke is forked and has a plurality of branches, each branch having one end connected to a trunk part of the elastic support spoke, and the other end fixed to the elastic expansion arm or the nasal passage engagement element.

As one of improvements of the above technical solution, the connecting beam applies a clipping force to the nasal septum, the clipping force being applied directly to the lower end of the nasal septum by the two connecting arms of the connecting beam, or after being conducted by the two connecting arms of the connecting beam to the corresponding expansion mechanisms, the clipping force being applied to the lower end of the nasal septum by the corresponding expansion mechanisms.

As one of improvements of the above technical solution, an average transverse cross-sectional area of the intermediate portion of the connecting beam is larger than that of the connecting arms.

As one of improvements of the above technical solution, the release element includes a medicine barrel, and a barrel lid and a barrel bottom connected to and covering two ends of the medicine barrel; a maximum diameter of the barrel lid is greater than a maximum diameter of the medicine barrel; a maximum diameter of the barrel bottom is greater than the maximum diameter of the medicine barrel; and
the medicine barrel is a hollow tubular structure, which contains a compound therein, and the barrel lid and the barrel bottom are provided with at least one opening to allow the compound to be discharged through the opening towards the nasal passage.

As one of improvements of the above technical solution, the medicine barrel is provided with an absorbent block made of a porous material therein.

As one of improvements of the above technical solution, the release element is made of a porous material.

As one of improvements of the above technical solution, an outer surface of the release element is provided with bulges.

Compared with the prior art, the arm, present invention has the following beneficial effects:
1. The expansion mechanism of the slow release apparatus of the present invention adopts a closed structure or an open structure, which improve the functionality and applicability of the slow release apparatus, and also improves the diversity of its specific implementations.
2. In addition to the nasal passage engagement element, the outer side primary surface of the elastic expansion arm can also be fit to the inner wall of the nasal passage, which increases the contact area between the expansion mechanism and the inner wall of the nasal passage, is conducive to expanding the nasal passage in all directions, and reduces the intensity of pressure of the expansion mechanism on the inner wall of the nasal passage and improves comfort.
3. The use of at least one elastic support spoke ensures the strength and balance of expansion and enables targeted expansion of specific parts of the nasal passage.
4. The release element does not contact the inner wall of the nasal passages, and thus avoid the compound contained therein contacting and irritating the inner wall of the nasal passage.
5. The release element adopts a column structure, a transverse cross-sectional area is small, so it has low air resistance, and by increasing the length of the release element along the axis of the nasal passage, its capacity can be effectively increased without increasing air resistance.
6. The release element is fixed in a simple and reliable way, which avoids a risk of escaping and being sucked in.
7. By replacing the release element with different ones, it can be adapted to release compounds of different states and types, in particular, to release non-volatile liquid compounds.
8. By replacing the release elements or their components with different ones, the speed of releasing a compound can be adjusted.

### BRIEF DESCRIPTION OF THE DRAWINGS

Fig. 1A is a side view of a human nasal part;
Fig. 1B is a side sectional view of a human nasal part;
Fig. 1C is a top view of a human nasal part from the bottom up;
Fig. 2 is a structural diagram of a first preferred embodiment of a slow release apparatus for slowly releasing a compound in a nasal passage and expanding the nasal passage of the present invention;
Fig. 3 is a top view of the first preferred embodiment of a slow release apparatus for slowly releasing a compound in a nasal passage and expanding the nasal passage of the present invention in Fig. 2;
Fig. 4 is a side view in a direction of the first preferred embodiment of a slow release apparatus for slowing releasing a compound in a nasal passage and expanding the nasal passage of the present invention in Fig. 2;
Fig. 5 is a side view in another direction of the first preferred embodiment of a slow release apparatus for slowing releasing a compound in a nasal passage and expanding the nasal passage of the present invention in Fig. 2;
Fig. 6 is a structural diagram of the first preferred embodiment of a slow release apparatus for slowing releasing a compound in a nasal passage and expanding the nasal passage of the present invention in Fig. 2 provided with an elastic support spoke;
Fig. 7 is a side sectional view of a release element in the first preferred embodiment of a slow release apparatus for slowing releasing a compound in a nasal passage and expanding the nasal passage of the present invention in Fig. 2;
Fig. 8 is a bottom-up top view of the first preferred embodiment of a slow release apparatus for slowing releasing a compound in a nasal passage and expanding the nasal passage of the present invention in Fig. 2 mounted to a nasal part of a user;
Fig. 9 is a side view of the first preferred embodiment of a slow release apparatus for slowing releasing a compound in a nasal passage and expanding the nasal passage of the present invention in Fig. 2 mounted to a nasal part of a user;
Fig. 10 is a structural diagram of a second preferred embodiment of a slow release apparatus for slowly releasing a compound in a nasal passage and expanding the nasal passage of the present invention;
Fig. 11 is a top view of the second preferred embodiment of a slow release apparatus for slowly releasing a compound in a nasal passage and expanding the nasal passage of the present invention in Fig. 10;
Fig. 12 is a structural diagram of the second preferred embodiment of a slow release apparatus for slowly releasing a compound in a nasal passage and expanding the nasal passage of the present invention in Fig. 10 provided with a forked elastic support spoke;
Fig. 13 is a side sectional view of a release element in the second preferred embodiment of a slow release apparatus for slowing releasing a compound in a nasal passage and expanding the nasal passage of the present invention in Fig. 10;
Fig. 14 is a structural diagram of a third preferred embodiment of a slow release apparatus for slowing releasing a compound in a nasal passage and expanding the nasal passage of the present invention;
Fig. 15 is a top view of the third preferred embodiment of a slow release apparatus for slowing releasing a compound in a nasal passage and expanding the nasal passage of the present invention in Fig. 14;
Fig. 16 is a side view of the third preferred embodiment of a slow release apparatus for slowing releasing a compound in a nasal passage and expanding the nasal passage of the present invention in Fig. 14;
Fig. 17A is a side sectional view of a release element in the third preferred embodiment of a slow release apparatus for releasing a compound in a nasal passage and expanding the nasal passage of the present invention in Fig. 14;
Fig. 17B is a side sectional view of a release element in the third preferred embodiment of a slow release apparatus for releasing a compound in a nasal passage and expanding the nasal passage of the present invention in Fig. 14, the release element being provided with protrusions at two ends;
Fig. 18A is a top view of a positional relationship between an elastic expansion arm and a nasal passage engagement element in other embodiments of a slow release apparatus for slowing releasing a compound in a nasal passage and expanding the nasal passage of the present invention; and
Fig. 18B is a top view of another positional relationship between an elastic expansion arm and a nasal passage engagement element in other embodiments of a slow release apparatus for slowing releasing a compound in a nasal passage and expanding the nasal passage of the present invention.

### Reference numerals:

| | | | |
|---|---|---|---|
| 1, | Slow release apparatus | 2, | Connecting beam |
| 3, | Elastic expansion arm | 4, | Nasal passage engagement element |
| 5, | fixing tube | 6, | Release element |
| 7, | Elastic support spoke | | |
| 11, | Forward direction of nasal part | 12, | Rearward direction of nasal part |
| 13, | Upward direction of nasal part | 14, | Downward direction of nasal part |
| 21, | First connecting beam | 22, | Second connecting beam |
| 23, | Intermediate portion | 24, | Connecting arm |
| 31, | Bent portion | 32, | Proximal arm |
| 33, | Distal arm | 34, | Inner side primary surface |
| 35, | Outer side primary surface | 36, | Hook |
| 41, | Inner primary surface | 42, | Outer primary surface |
| 61, | Medicine barrel | 62, | barrel lid |
| 63, | Barrel bottom | 64, | Content |
| 65, | Bulge | | |
| 71, | First elastic support spoke | 72, | Second elastic support spoke |
| 73, | First branch | 74, | Second branch |
| 75, | Third branch | 76, | Fourth branch |
| 77, | Jack | | |
| 91, | Nasal septum | 92, | Nasal wing |
| 93, | Nasal bridge | 94, | Nostril |
| 95, | Nasal passage | 96, | Inner wall of nasal passage |
| 97, | Nasal passage axis | 98, | Nasal cavity |
| 99, | Nasal base | | |
| 213, | First intermediate portion | 214, | First connecting arm |
| 223, | Second intermediate portion | 224, | Second connecting arm |
| 241, | Widened portion | 242, | Protrusion |
| 243, | Plug | | |
| 611, | Screw port | 612, | Clamping hole |
| 621, | First hole | 622, | Columnar protrusion |
| 623, | Thread | 624, | Hemispherical protrusion |
| 631, | Second hole | 641, | Absorbent block |
| 642, | Solid compound | | |
| 2431, | Cap | 2432, | Column |
| 2433, | Shank | | |

### DETAILED DESCRIPTION

Now, the present invention is further described in conjunction with the accompanying drawings.

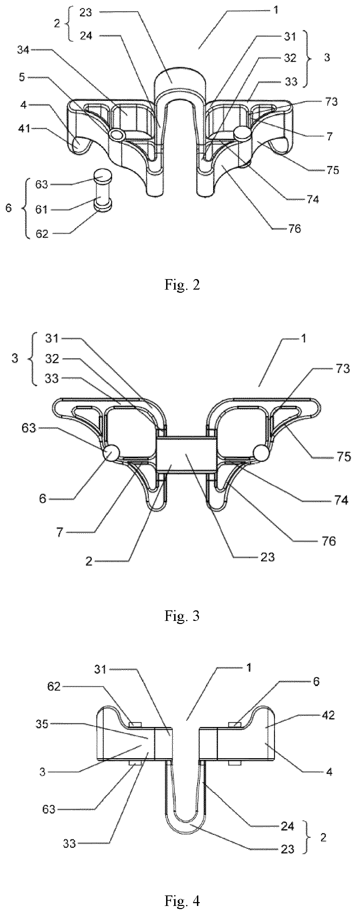

As shown in Figs. 2, 10 and 14, in a slow release apparatus 1 of the present invention, except for a connecting beam 2, other components are symmetrically arranged in two nasal passages 95. Therefore, the slow release apparatus 1 is described in the present invention with the numbers and structures of other components located in a nasal passage, except for the connecting beam 2.

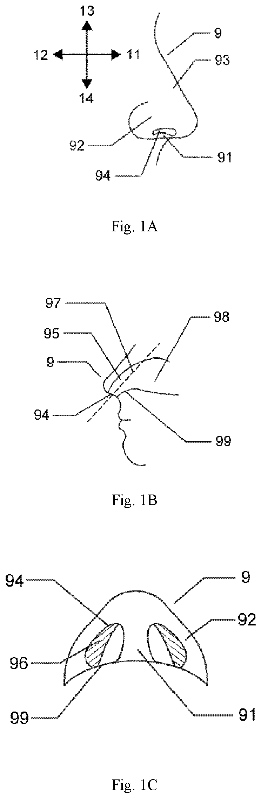

As shown in Fig. 1A, directions for a nasal part described in the present invention are: a forward direction 11 of the nasal part, a rearward direction 12 of the nasal part, an upward direction 13 of the nasal part, and a downward direction 14 of the nasal part.

As shown in Fig. 1B, in a nasal passage 95, a side of an object close to a nasal passage axis 97 is an "inner side", and a side away from the nasal passage axis 97 is an "outer side".

The term "connection" as used in the present invention means that two tangible objects are integrally connected together, or are detachably connected together by means of a connector, and connection modes include but are not limited to: adhesive bonding, fusion splicing, plug-in connection, screw connection, and snap connection.

The term "axis" as used in the present invention refers to the axis of centrosymmetry of a tube or column. For a non-standard tube or column, after it is approximated as a standard tube or column, an axis of the standard tube or column can be used as its approximate axis. For example, Fig. 1B depicts the nasal passage axis 97.

The term "transverse cross-section" as used in the present invention refers to a cross-section of the tube or column perpendicular to its axis.

The term "compound" as used in the present invention is not strictly defined chemically, but shall include pure substances, compounds and mixtures in solid and liquid forms, and other forms or combinations of substances applicable to the present invention. The compound may be pharmaceutical or non-pharmaceutical, including but not limited to drugs and aromatic substances.

The term "closed structure" as used in the present invention is a closed planar figure consisting of n (n is a positive integer) line segments or arcs, for example: a circle, an ellipse, or a polygon; and the term "open structure" refers to a planar figure formed by providing an opening in a closed figure, for example: a C, V, 1, or arc shape.

The term "tubular structure" as used in the present invention refers to a columnar structure provided internally with a cavity running therethrough along an axis direction, the columnar structure including, but not limited to, a cylinder and a prism.

As shown in Figs. 1A, 1B and 1C, the slow release apparatus 1 of the present invention is applied to a human nasal part 9. The nasal part 9 is a visually visible facial protruding part, which includes: a nasal septum 91, nasal wings 92, a nasal bridge 93 and a nasal base 99, and two nasal passages 95 enclosed thereby. Outlets of the two nasal passages 95 are two nostrils 94. An inner wall 96 of a nasal passage is composed of inner side surfaces of the parts enclosing the nasal passage 95 as described above. In the present invention, a nasal passage 95 is limited to a nasal vestibule contained by the nasal part 9 and does not include a nasal cavity 98.

As shown in Fig. 2, the present invention provides a slow release apparatus 1 for slowly releasing a compound in a nasal passage 95 and expanding the nasal passage 95, including: a connection mechanism, two expansion mechanisms, and two release mechanisms.

Two ends of the connection mechanism are respectively integrally or detachably connected to the two expansion mechanisms symmetrically arranged in nasal passages 95 at two sides. The two release mechanisms are symmetrically arranged, and each release mechanism is correspondingly provided in one of the expansion mechanisms.

The expansion mechanism is configured to expand the nasal passage 95 and the releasing mechanism is configured to contain and release a compound.

As shown in Figs. 2, 4, 5 and 9, the connection mechanism includes at least one connecting beam 2. The connecting beam 2 semi-surrounds a lower end of a nasal septum 91 and includes an intermediate portion 23 located on a lower side of the nasal septum 91 and two connecting arms 24 symmetrically arranged on two sides of the nasal septum 91. Two ends of the intermediate portion 23 are respectively connected to the two connecting arms 24. The two connecting arms 24 of the connecting beam 2 are inserted into the two nasal passages 95 from the two sides of the nasal septum 91, and the two connecting arms 24 are respectively connected to the two expansion mechanisms symmetrically arranged in the two nasal passages 95. Preferably, the connecting beam 2 is a U-shaped or C-shaped structure, wherein the C-shaped structure is more conducive for ends of the connecting arms 24 to apply a clipping force to the nasal septum 91.

Preferably, the connecting beam 2 also provides a fixing function for the slow release apparatus 1. A minimum distance between its two connecting arms 24 is less than a width of the nasal septum 91 there, so that they apply a clipping force to the nasal septum 91. The clipping force is applied to the two sides of the nasal septum 91 directly by the two connecting arms 24 of the connecting beam 2, or is conducted by the two connecting arms 24 of the connecting beam 2 to the corresponding expansion mechanisms and then applied to the two sides of the nasal septum 91 by the elastic expansion arms 3. The above-mentioned clipping force enables the slow release apparatus 1 to clip and be fixed to the nasal septum 91.

As shown in Figs. 2, 4 and 5, preferably, an average transverse cross-sectional area of the intermediate portion 23 of the connecting beam 2 is larger than that of the connecting arms 24, so that the intermediate portion 23 is thicker than the connecting arms 24, thereby strengthening the ability of the connecting beam 2 to maintain its own shape, thus improving its ability to apply a clipping force to the nasal septum 91.

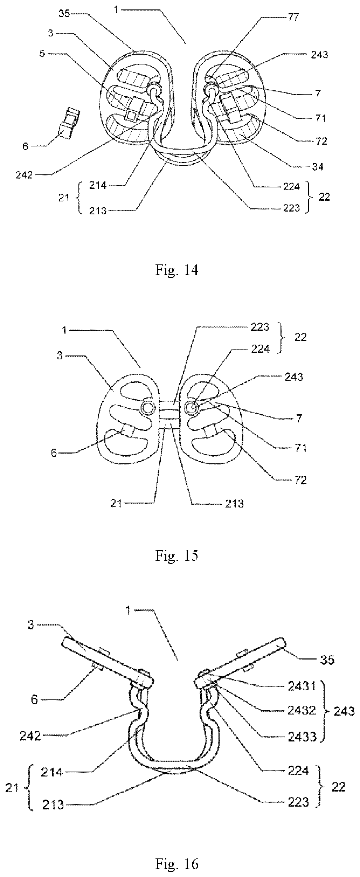

As shown in Figs. 14, 15 and 16, in other specific embodiments, the connecting beam 2 in the connection mechanism of the slow release apparatus 1 includes: a first connecting beam 21 and a second connecting beam 22. Since the first connecting beam 21 is integrally connected to and made of the same material as the expansion mechanisms, which material does not support the first connecting beam 21 to provide a sufficient clipping force, thus the second connecting beam 22 is also provided at a position longitudinally side by side with the first connecting beam 21, and the second connecting beam 22 is used to supplement the clipping force on the nasal septum 91.

As shown in Figs. 14 and 16, in other specific embodiments, the two second connecting arms 24 of the second connecting beam 2 are provided with protrusions 242 towards the nasal septum 91. The protrusions 242 are fit to the two sides of the nasal septum 91 and apply a clipping force to the nasal septum. In the case the protrusions 242 provide clipping and fixing functions, tail ends of the two connecting arms 24 of the connecting beam 2 can be connected to the expansion mechanisms at positions deeper in the nasal passages 95, thereby being conducive to arranging the expansion mechanisms and the release mechanisms at positions deeper in the nasal passages 95.

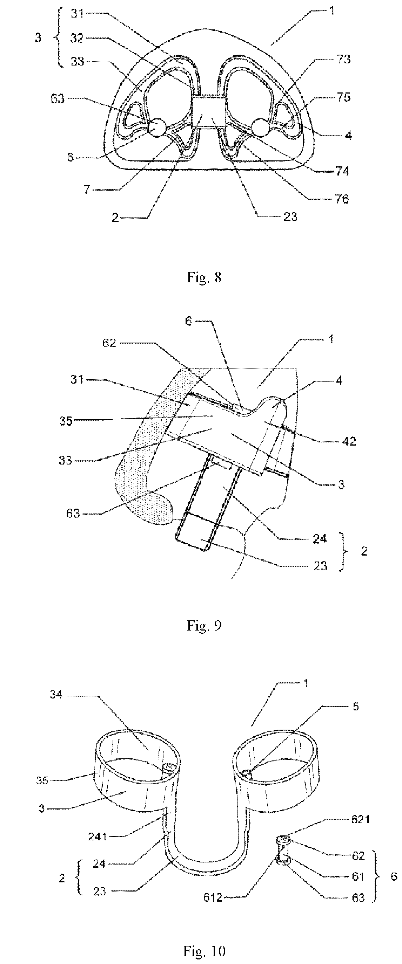

As shown in Fig. 10, in other specific embodiments, the two connecting arms 24 are provided with widened portions 241, and the connection mechanism applies a clipping force to the nasal septum 91 through the widened portions 241; and the width of a surface on a side of each connecting arm 24 facing towards the nasal septum 91 increases at the widened portion 241 to increase the fitting area of the widened portion 241 to the nasal septum 91, thereby reducing the intensity of pressure of the connecting beam 2 on the nasal septum 91 to improve comfort.

In the case the connecting beam 2 is provided with both widened portions 241 and protrusions 242, they can be combined into same parts, which have features of both the widened portions 241 and the protrusions 242.

As shown in Figs. 14, 15 and 16, in other specific embodiments, ends of the two connecting arms 24 of the connecting beam 2 are provided with plugs 243. Each plug 243 is a structure wide at two ends and narrow in the middle, and fixes the connecting beam 2 to the expansion mechanism in a plug-in manner.

Specifically, as shown in Fig. 16, the plug 243 is inserted into a jack 77 provided in the elastic expansion arm 3 or an elastic support spoke 7 to achieve plug-in fixation. The plug 243 is a structure wide at two ends and narrow in the middle, including a cap 2431, a column 2432 and a shank 2433. The jack 77 is tightly engaged with the column 2432. A maximum width of the shank 2433 is greater than a maximum width of the column 2432, and a maximum width of the cap 2431 is greater than the maximum width of the column 2432; also, the maximum width of the shank 2433 is greater than a maximum width of an inner cavity of the jack 77, and the maximum width of the cap 2431 is greater than the maximum width of the inner cavity of the jack 77. In this way, the plug 243 cannot escape. Preferably, the column 2432 and the inner cavity of the jack 77 can be configured to be prismatic, to avoid rotation of the expansion mechanism around the plug 243.

As shown in Fig. 2, the two expansion mechanisms are symmetrically connected to the connection mechanism, and each expansion mechanism includes an elastic expansion arm 3, a nasal passage engagement element 4 and at least one elastic support spoke 7.

As shown in Figs. 2, 4, 5 and 9, the nasal passage engagement element 4 is a sheet-like structure, which is connected to the elastic expansion arm 3, and when worn, the nasal passage engagement element 4 is located on an inner side of a nasal wing 92 and applies an expansion force to the nasal wing 92. A pair of surfaces of the nasal passage engagement element 4 parallel to the nasal passage axis 97 are its primary surfaces and largest surfaces. The pair of primary surfaces are specifically an inner primary surface 41 and an outer primary surface 42, each having a width generally greater than that of a surface on the same side of the elastic expansion arm 3. The outer primary surface 42 is fit to an inner wall of the nasal wing 92 and applies an expansion force from the elastic expansion arm 3 and the elastic support spoke 7 to the nasal wing 92. A surface of the nasal passage engagement element 4 perpendicular to the nasal passage axis 97 is its secondary surface, the width of which is less than the widths of the primary surfaces to reduce respiratory resistance.

As shown in Figs. 2, 3, 4, 6 and 8, the elastic expansion arm 3 is an elastically compressible open band-like structure, such as a C-shaped, V-shaped, 1-shaped or arc-shaped band-like structure, which is connected to the connecting arm 24 and the nasal passage engagement element 4. A pair of surfaces of the elastic expansion arm 3 parallel to the nasal passage axis 97 are its primary surfaces and largest surfaces. The pair of primary surfaces are specifically an inner side primary surface 34 and an outer side primary surface 35. A surface of the elastic expansion arm 3 perpendicular to the nasal passage axis 97 is its secondary surface, the width of which is smaller than the widths of the primary surfaces to reduce respiratory resistance. As shown in Fig. 8, the outer side primary surface 35 of the elastic expansion arm 3 partially or entirely is fit to the inner wall 96 of the nasal passage, so as to be capable of applying an expansion force from the elastic expansion arm 3 and the elastic support spoke 7 to the nasal wing 92 and applying a clipping force from the connecting beam 2 to the nasal septum 91. Since the elastic expansion arm 3 is an open structure, it is more adaptable to nasal passages of different shapes and sizes, and is particularly suitable for users whose left and right nasal passages have different sizes due to deviation of nasal septum, thus improving user experience and comfort and conforming to ergonomic design.

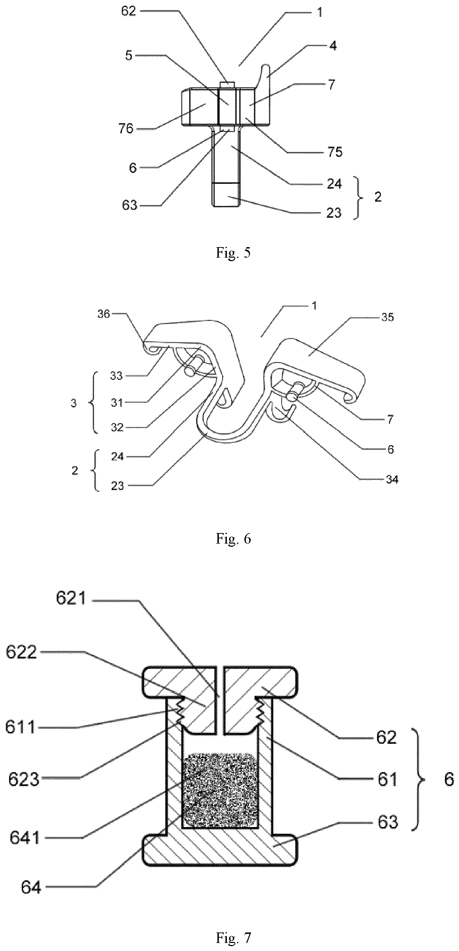

As shown in Fig. 6, if the width of the nasal passage engagement element 4 is equal to or smaller than that of the elastic expansion arm 3, and their primary surfaces on a same side are smoothly connected and fused into a same surface, i.e., the inner side primary surface 34 and the inner primary surface 41 are fused into one surface, and the outer side primary surface 35 and the outer primary surface 42 are fused into one surface, in this case, the elastic expansion arm 3 and the nasal passage engagement element 4 are fused into an integral whole, and the function of the nasal passage engagement element 4 is completely replaced by the elastic expansion arm 3, and in this case, the expansion mechanism does not include the nasal passage engagement element 4, and only includes the elastic expansion arm 3 and the elastic support spoke 7.

Preferably, an end of the elastic expansion arm 3, at a part fit to the inner wall 96 of the nasal passage, is bent towards an inner side of the nasal passage 95; for example, as shown in Fig. 6, tail ends of a proximal arm 32 and a distal arm 33 of the elastic expansion arm 3 are hooks 36, and the hooks 36 can flexibly contact the inner wall 96 of the nasal passage; and an end of the nasal passage engagement element 4 is bent towards the inner side of the nasal passage 95.

Preferably, as shown in Figs. 2, 3, 4, 6 and 8, a transverse cross-section of the elastic expansion arm 3 perpendicular to the nasal passage axis 97 is a V-shaped structure or C-shaped structure including a bent portion 31, a proximal arm 32 and a distal arm 33.

The bent portion 31 is connected to the proximal arm 32 at one end and to the distal arm 31 at the other end. When worn, the proximal arm 32 is close to the nasal septum 91, the distal arm 33 is close to the nasal wing 92, and the bent portion 31 is close to the nasal bridge 93 or the nasal base 99, that is, an opening of the elastic expansion arm 3 may face the nasal bridge 93, and may also face the nasal base 99. The outer side primary surface 35 of the elastic expansion arm 3 is fit to the inner wall 96 of the nasal passage. As shown in Fig. 8. when worn, the elastic expansion arm 3 is compressed by the inner wall 96 of the nasal passage such that the distal arm 33 and the proximal arm 32 get closer to each other, and a resilience force of the bent portion 31 causes the distal arm 33 and the nasal passage engagement element 4 to apply an expansion force to the nasal wing 92, thereby expanding the nasal passage, and at the same time, the elastic expansion arm 3 and the nasal passage engagement element 4 are tightly fit to the inner wall 96 of the nasal passage, and their interaction forces fix the slow release apparatus 1 in the nasal passage 95.

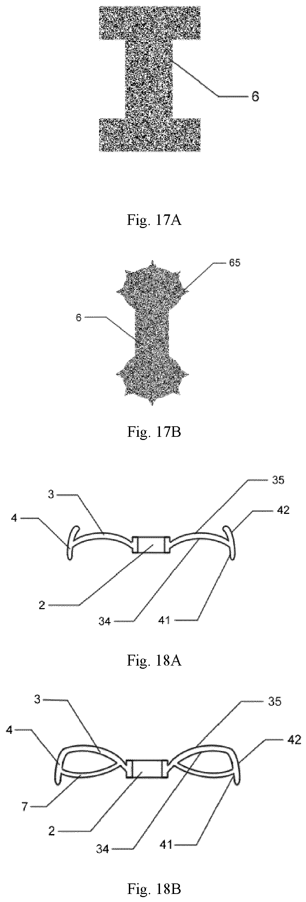

As shown in Fig. 18A, in other specific embodiments, the elastic expansion arm 3 is an arc-shaped or I-shaped open band-like structure, one end of which is connected to the end of the connecting arm 24 and the other end of which is connected to the inner primary surface 41 of the nasal passage engagement element 4, and the elastic expansion arm 3 is not fused with the nasal passage engagement element 4 into an integral whole. When worn, the outer primary surface 42 of the nasal passage engagement element 4 is fit to the inner wall 96 of the nasal passage, but the elastic expansion arm 3 are not fit to the inner wall 96 of the nasal passage. In addition, since the expansion mechanism is fit to the inner wall 96 of the nasal passage in a small area, no elastic support spoke is provided to provide multi-point support.

As shown in Fig. 18B, in other specific embodiments, the elastic expansion arm 3 is an arc-shaped or I-shaped open band-like structure, one end of which is connected to the end of the connecting arm 24 and the other end of which is connected to the secondary surface on one side of the nasal passage engagement element 4. When worn, the outer primary surface 42 of the nasal passage engagement element 4 is fit to the inner wall 96 of the nasal passage, but the elastic expansion arm 3 is not fit to the inner wall 96 of the nasal passage. Since the elastic expansion arm 3 is not smoothly connected to a corresponding primary surface of the nasal passage engagement element 4 and does not form one surface therewith, they are not fused into an integral whole.

As shown in Fig. 6, the elastic support spoke 7 is an elastically compressible band-like or strip-like structure, two ends of which are connected to the elastic expansion arm 3 or the nasal passage engagement element 4. When worn, the elastic support spoke 7 applies a resilience force generated after being compressed to the elastic expansion arm 3 and/or the nasal passage engagement element 4. The elastic support spoke 7 can increase the expansion force of the expansion mechanism, and also improves the balance of expansion. Since the elastic expansion arm 3 is an open structure, its resilience force is weaker at positions closer to its ends. The elastic support spoke 7 can provide targeted support at specific positions of the elastic expansion arm 3 and the nasal passage engagement element 4, thus ensuring the balance of expansion of the nasal passage 95 by the expansion mechanism.

As shown in Figs. 2, 3 and 8, preferably, the elastic support spoke 7 is forked and includes a plurality of branches, specifically: a first branch 73, a second branch 74, a third branch 75 and a fourth branch 76, each branch having one end connected to a trunk part of the elastic support spoke 7, and the other end fixed to the elastic expansion arm 3 or the nasal passage engagement element 4. The plurality of branches of the elastic support spoke 7 enable it to have stronger resilience, thus further increasing the expansion strength, and its plurality of branches also provides more support points, which also further improves the targeting and balance of expansion.

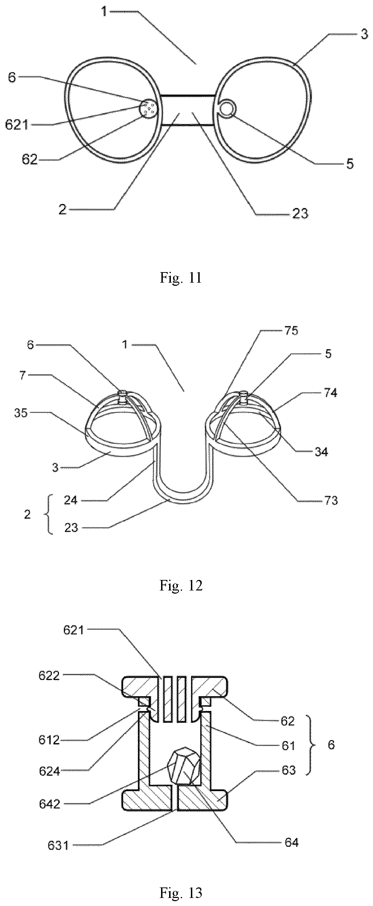

As shown in Figs. 10 and 11, in other specific embodiments, the expansion mechanism includes an elastic expansion arm 3. The elastic expansion arm 3 is a ring-like closed structure, and its outer side primary surface 35 is fit to the inner wall 96 of the nasal passage. When worn, the elastic expansion arm 3 is compressed by the inner wall 96 of the nasal passage, and its resilience force causes it to apply an expansion force in various directions to the nasal passage 95. At the same time, the elastic expansion arm 3 is tightly fit to the inner wall 96 of the nasal passage, and their interaction forces fix the slow release apparatus 1 in the nasal passage 95. The ring-like elastic expansion arm 3 can expand the nasal passage in all directions and is capable of increasing the contact area between the expansion mechanism and the inner wall 96 of the nasal passage, thereby reducing the intensity of pressure of the expansion mechanism on the inner wall 96 of the nasal passage to improve comfort.

As shown in Fig. 12, in other specific embodiments, the expansion mechanism includes an elastic expansion arm 3 and at least one elastic support spoke 7. The elastic expansion arm 3 is a ring-like structure, and the elastic support spoke 7 is forked and includes a plurality of branches, specifically: a first branch 73, a second branch 74 and a third branch 75, each branch having one end connected to a trunk part of the elastic support spoke 7, and the other end fixed to the elastic expansion arm 3. When worn, the elastic support spoke 7 applies a resilience force generated after being compressed to the elastic expansion arm 3. Preferably, the elastic support spoke 7 and a fixing tube 5 and a release element 6 fixed to the elastic support spokes 7 are located above the elastic expansion arm 3, forming an inverted bowl-shaped structure, which is conducive to releasing the compound deeper into a respiratory tract by the release mechanism.

As shown in Figs. 14 and 15, in other specific embodiments, the expansion mechanism includes an elastic expansion arm 3 and at least one elastic support spoke 7. The elastic expansion arm 3 is a ring-like closed structure, and the elastic support spoke 7 is an elastically compressible band-like or strip-like structure, two ends of which are connected to the elastic expansion arm 3.

As shown in Fig. 2, the two release mechanisms are symmetrically arranged, and each release mechanism is correspondingly provided in one of the expansion mechanisms, each release mechanism including at least one fixing tube 5 and at least one release element 6.

As shown in Fig. 2, the fixing tube 5 is a tubular structure, which is used to fix the release element 6, and the fixing tube 5 is provided in the direction of the opening of the elastic expansion arm 3, and the fixing tube 5 is fixed to a trunk part of the elastic support spoke 7 by means of a first branch 73, a second branch 74, a third branch 75 and a fourth branch 76. As shown in Figs. 10 and 14, in other specific embodiments, the fixing tube 5 is fixed to an inner side of the elastic expansion arm 3 or an inner side of the nasal passage engagement element 4. Specifically, a fixing tube 5 shown in Fig. 10 is fixed to an inner side of a ring-like elastic expansion arm 3. As shown in Fig. 14, in other specific embodiments, a quadrangular-prism fixing tube 5 is fixed to a second elastic support spoke 72. As an optimization, the slow release apparatus 1 is provided with a plurality of fixing tubes 5 and release elements 6 to increase a capacity for the compound or increase a release speed of the compound.

As shown in Figs. 2, 10 and 14, the release element 6 is a structure wide at two ends and narrow in the middle. When worn, the release element 6 is fixed to the fixing tube 5, the fixing tube 5 surrounds the thinner middle of the release element 6 and is tightly engaged therewith, and the two ends of the release element are located on outer sides of two ends of the fixing tube 5, with their maximum widths being greater than a maximum width of an inner cavity of the fixing tube 5, so that the release element 6 cannot escape from the fixing tube 5. The release element 6 is capable of containing a certain amount of compound and allows the compound to be slowly released in the nasal passage 95. When worn, at least part of the release element 6 is located in the nasal passage 95.

The release mechanism has the following advantages: First, the fixing method is simple and reliable; second, the area of the transverse cross-section is small and the respiratory resistance is low; third, its length and capacity can be increased in the direction of the nasal passage axis as needed without increasing the respiratory resistance; fourth, by replacing the release elements 6 with those of different types, it can be adapted to different compounds or provide different release speeds; fifth, the compound contained in the release element 6 can be replaced conveniently; and sixth, the release speed of the compound can be adjusted by adjusting the number and size of openings of the release element 6.

As shown in Figs. 14, 17A and 17B, in other specific embodiments, the material of the release element 6 is a porous material for absorbing and holding a liquid compound. Its porous surface is particularly conducive to compound volatilization, and also conducive to an inspiratory airflow in the nasal passage carrying away tiny compound droplets, so the porous material is not only capable of releasing volatile liquid compounds, but also capable of releasing non-volatile liquid compounds in the form of tiny droplets. As shown in Fig. 17B, as an optimization, surfaces of two ends of the release element 6 made of the porous material are provided with bulges 65 to further increase its surface area and increase the release speed.

As shown in Figs. 2, 7, 10 and 13, in other specific embodiments, the release element 6 includes: a medicine barrel 61, and a barrel lid 62 and a barrel bottom 63 connected to and covering two ends of the medicine barrel 61.

Maximum widths of the barrel lid 62 and the barrel bottom 63 are greater than a maximum width of the medicine barrel 61. The medicine barrel 61 is a hollow tubular structure, the interior of which is used to contain solid and liquid compounds, and the barrel lid 62 and the barrel bottom 63 are at least provided with one opening to allow the compound to be discharged through the opening. As shown in Fig. 7, as an optimization, the medicine barrel 61 is provided with an absorbent block 641 made of a porous material therein, which is used to absorb and contain a liquid compound to avoid the liquid compound from flowing out of the opening, and also capable of increasing the release speed of the compound by its porous surface.

### Embodiment 1.

Figs. 2, 3, 4, 5, 6, 7, 8 and 9 illustrate a first preferred embodiment of the present invention, in which the slow release apparatus 1 includes: a connection mechanism, two expansion mechanisms and two release mechanisms.

As shown in Figs. 2, 4, 5 and 9, the connection mechanism includes a connecting beam 2 of a U-shaped structure. The connecting beam 2 semi-surrounds a lower end of a nasal septum 91, as shown in Fig. 2, the connecting beam 2 including an intermediate portion 23 located on a lower side of the nasal septum 91 and two connecting arms 24 symmetrically arranged on two sides of the nasal septum 91. Two ends of the intermediate portion 23 are respectively connected to the two connecting arms 24. When worn, the two connecting arms 24 are inserted into two nasal passages 95 from the two sides of the nasal septum 91, and are respectively integrally connected to two elastic expansion arms 3 symmetrically arranged in the two nasal passages 95. A minimum distance between the two connecting arms 24 is less than a width of the nasal septum 91 there, so that they apply a clipping force to the nasal septum 91, thereby fixing the slow release apparatus 1 to the nasal septum 91. An average transverse cross-sectional area of the intermediate portion 23 is larger than that of the connecting arms 24, so that the intermediate portion 23 is thicker than the connecting arms 24, to improve the ability of the connecting beam 2 to maintain its own shape, thus strengthen the clipping force.

As an optimization, the two connecting arms 24 of the connecting beam 2 can be symmetrically provided with widened portions 241, and the width of a surface on a side of each connecting arm 24 facing towards the nasal septum 91 increases at the widened portion 241. The two widened portions 241 are fit to two sides of the nasal septum 91 and apply a clipping force to the nasal septum 91.

As an optimization, the two connecting arms 24 of the connecting beam 2 can be symmetrically provided with protrusions 242 towards the nasal septum 91 of a wearer. The protrusions 242 are fit to the two sides of the nasal septum 91 and apply a clipping force to the nasal septum 91.

As an optimization, ends of the two connecting arms 24 of the connecting beam 2 can be provided with plugs 243. The plugs 243 are inserted into jacks 77 provided in the elastic expansion arms 3 or support spokes. Each plug 243 is a structure wide at two ends and narrow in the middle to achieve plug-in fixation.

As shown in Fig. 2, the two expansion mechanisms are symmetrically connected to the connection mechanism, and each expansion mechanism includes an elastic expansion arm 3, a nasal passage engagement element 4 and an elastic support spoke 7.

As shown in Figs. 2, 4, 5 and 9, the nasal passage engagement element 4 is a sheet-like structure, which is located on an inner side of a nasal wing 92 of a wearer. The nasal passage engagement element 4 is connected to an end of a distal arm 33 of the elastic expansion arm 3, and a pair of surfaces thereof parallel to a nasal passage axis 97 are its primary surfaces and largest surfaces. The pair of primary surfaces are an inner primary surface 41 and an outer primary surface 42. The outer primary surface 42 is fit to an inner wall of the nasal wing 92, and thereby applies an expansion force from the elastic expansion arm 3 and the elastic support spoke 7 to the nasal wing 92. The widths of the primary surfaces of the nasal passage engagement element 4 are greater than that of a primary surface of the distal arm 33, thereby better dispersing the intensity of expansion pressure of the expansion mechanism on the nasal wing 92 to improve comfort. A surface of the nasal passage engagement element 4 perpendicular to the nasal passage axis 97 is its secondary surface, the width of which is less than the widths of the primary surfaces to reduce respiratory resistance.

As an optimization, a tail end of the nasal passage engagement element 4 can be bent toward an inner side of the nasal passage 95 to avoid irritation of an inner wall 96 of the nasal passage by its tail end.

As shown in Figs. 2 and 3, the elastic expansion arm 3 is connected to the connecting arm 24 and the nasal passage engagement element 4, and its transverse cross-section perpendicular to the nasal passage axis 97 is a V-shaped structure. The elastic expansion arm 3 includes a bent portion 31, a proximal arm 32 and a distal arm 33. The bent portion 31 is connected to the proximal arm 32 at one end and to the distal arm 33 at the other end. When worn, the proximal arm 32 is close to the nasal septum 91, the distal arm 33 is close to the nasal wing 92, and the bent portion 31 is close to the nasal bridge 93, and as shown in Figs. 1C and 8, at that time, an opening of the elastic expansion arm 3 faces a nasal base 99. As an optimization, the proximal arm 32 of the elastic expansion arm 3 is close to the nasal septum 91, the distal arm 33 is close to the nasal wing 92, and the bent portion 31 is close to the nasal base 99, such that the opening of the elastic expansion arm 3 faces the nasal bridge 93. A pair of surfaces of the elastic expansion arm 3 parallel to the nasal passage axis 97 are its primary surfaces and largest surfaces. The pair of primary surfaces are an inner side primary surface 34 and an outer side primary surface 35. A surface of the elastic expansion arm 3 perpendicular to the nasal passage axis 97 is its secondary surface, the width of which is smaller than the widths of the primary surfaces to reduce air resistance. The outer side primary surface 35 of the elastic expansion arm 3 entirely is fit to the inner wall 96 of the nasal passage, so as to be capable of applying an expansion force from the elastic expansion arm 3 and the elastic support spoke 7 to the nasal wing 92.

As shown in Fig. 8. when worn, the elastic expansion arm 3 is compressed by the inner wall 96 of the nasal passage such that the distal arm 33 and the proximal arm 32 get closer to each other, and a resilience force of the bent portion 31 causes the distal arm 33 and the nasal passage engagement element 4 to apply an expansion force to the nasal wing 92, thereby expanding the nasal passage, and at the same time, the elastic expansion arm 3 and the nasal passage engagement element 4 are tightly fit to the inner wall 96 of the nasal passage, and their interaction forces fix the slow release apparatus 1 in the nasal passage 95. Since the elastic expansion arm 3 is an open structure, it is more adaptable to nasal passages of different shapes and sizes, and is particularly suitable for users whose left and right nasal passages have different sizes due to deviation of nasal septum.

As shown in Fig. 6, if the width of the nasal passage engagement element 4 is equal to or smaller than that of the elastic expansion arm 3, and their primary surfaces on a same side are smoothly connected and fused into a same surface, i.e., the inner side primary surface 34 and the inner primary surface 41 are fused into one surface, and the outer side primary surface 35 and the outer primary surface 42 are fused into one surface, in this case, the elastic expansion arm 3 and the nasal passage engagement element 4 are fused into an integral whole, and both the elastic expansion arm 3 and the nasal passage engagement element 4 are fit to the inner wall 96 of the nasal passage, and their primary surfaces are actually the same surface, and thus can be considered as fusion. The function of the nasal passage engagement element 4 is completely replaced by the elastic expansion arm 3, and in this case, the expansion mechanism does not include the nasal passage engagement element 4, and only includes the elastic expansion arm 3 and the elastic support spoke 7.

As shown in Fig. 6, as an optimization, tail ends of the proximal arm 32 and the distal arm 33 of the elastic expansion arm 3 are bent toward the inner side of the nasal passage 95 to form hooks 36 to avoid irritation of the inner wall of the nasal passage by their tail ends.

As shown in Figs. 2, 3 and 8, the expansion mechanism has a forked elastic support spoke 7, which includes a first branch 73, a second branch 74, a third branch 75 and a fourth branch 76, wherein the first branch 73 is connected to the distal arm 33 of the elastic expansion arm 3, the second branch 74 and the fourth branch 76 are connected to the proximal arm 32, and the third branch 75 is connected to the nasal passage engagement element 4. When worn, the elastic support spoke 7 applies a resilience force generated after being compressed to the elastic expansion arm 3 and the nasal passage engagement element 4. The plurality of branches of the elastic support spoke 7 enable it to have stronger resilience, thus further improving an expansion effect, and its plurality of branches increase support points for the elastic expansion arm 3 and the nasal passage engagement element 4, thus further improving the targeting and balance of expansion.

Fig. 6 depicts a first preferred embodiment in which an elastic support spoke 7 is provided. The elastic support spoke 7 is an arc-shaped band-like structure, two ends of which are connected to different positions of the inner side primary surface 34 of the elastic expansion arm 3. The band-like elastic support spoke 7 can improve the expansion effect and increase the targeting and balance of expansion, and also has the characteristics of a simple structure and low air resistance. As an optimization, a plurality of band-like elastic support spokes 7 can be provided as need actually to further improve expansion strength and balance.

As shown in Fig. 2, the two release mechanisms are symmetrically arranged, and each release mechanism is correspondingly provided in one of the expansion mechanisms, each release mechanism including a fixing tube 5 and a release element 6.

As shown in Fig. 2, the fixing tube 5 is a circular tubular structure, which is fixed to the elastic support spokes 7, and the fixing tube 5 is used to surround and fix the release element 6. As an optimization, the fixing tube 5 can also be provided on the inner side primary surface 34 of the elastic expansion arm 3, or on the inner primary surface 41 of the nasal passage engagement element 4.

As shown in Figs. 2 and 7, the release element 6 is a structure wide at two ends and narrow in the middle, which includes a hollow circular tubular medicine barrel 61 and a barrel lid 62 and a barrel bottom 63 connected to and covering two ends of the medicine barrel.

Outer diameters of the barrel lid 62 and the barrel bottom 63 are greater than an outer diameter of the medicine barrel 61. When worn, the release element 6 is fixed to the fixing tube 5, the fixing tube 5 surrounds the medicine barrel 61 and is tightly engaged therewith, and the barrel lid 62 and the barrel bottom 63 are located on outer sides of two ends of the fixing tube 5, with their outer diameters being greater than an inner diameter of the fixing tube 5, so that the release element 6 cannot escape from the fixing tube 5, and at that time, the release element 6 is located in the nasal passage 95. The barrel lid 62 is provided with a columnar protrusion 622, which is provided with a thread 623 at an outer side. The columnar protrusion 622 is screwed into an open end of the medicine barrel 61 such that the thread 623 is tightly engaged with a screw port 611 at the open end of the medicine barrel 61 to achieve threaded fit, thereby connecting the medicine barrel 61 and the barrel lid 62. The barrel bottom 63 and the medicine barrel 61 are an integrated structure. The medicine barrel 61 is used to contain solid and liquid compounds, and the barrel lid 62 is provided with a first hole 621 to allow the compound to be released to the nasal passage through the first hole 621. Contents 64 of the medicine barrel 61 are an absorbent block 641 made of a porous material and a liquid compound absorbed therein. The absorbent block 641 can prevent the liquid compound from flowing out of the first hole 621, and its porous surface can also increase the release speed of the compound. The release mechanism allows convenient replacement of the contents 64 of the medicine barrel, and also allows replacement of the barrel lid 62 with one having a different number of openings of a different size, to adjust the release speed, and the release element 6 can also be replaced as a whole with a porous material to accommodate to different release needs.

### Embodiment 2.

Figs. 10, 11, 12 and 13 illustrate a second preferred embodiment of the present invention, in which the slow release apparatus 1 includes: a connection mechanism, two expansion mechanisms and two release mechanisms.

As shown in Figs. 10 and 11, the connection mechanism includes a connecting beam 2 of a U-shaped structure. The connecting beam 2 semi-surrounds a lower end of a nasal septum 91, and the connecting beam 2 includes an intermediate portion 23 located on a lower side of the nasal septum 91 and two connecting arms 24 symmetrically arranged on two sides of the nasal septum 91. The two connecting arms 24 of the connecting beam 2 are inserted into two nasal passages 95 from the two sides of the nasal septum 91, and are respectively integrally connected to two ring-like elastic expansion arms 3 symmetrically arranged in the two nasal passages 95.

The connecting beam 2 also provides a fixing function for the slow release apparatus 1. A minimum distance between its two connecting arms 24 is less than a width of the nasal septum 91 there, so that they apply a clipping force to the nasal septum 91, thereby enabling the slow release apparatus 1 to clip and be fixed to the nasal septum 91. Parts of the connecting beam 2 that apply the clipping force to the nasal septum 91 are widened portions 241, and surfaces of the connecting arms 24 facing towards the nasal septum 91 are wider at the widened portions 241 to increase the fitting area of the widened portions 241 and the nasal septum 91, thereby reducing the intensity of pressure of the widened portions 241 on the nasal septum 91 to improve comfort.

As an optimization, the two connecting arms 24 of the connecting beam 2 can be symmetrically provided with protrusions 242 towards the nasal septum 91 of a wearer. The protrusions 242 are fit to the two sides of the nasal septum 91 and apply a clipping force to the nasal septum 91. The protrusions 242 can be combined with the widened portions 241 into same parts.

As an optimization, ends of the two connecting arms 24 of the connecting beam 2 can be provided with plugs 243. The plugs 243 are inserted into jacks 77 provided in the elastic expansion arms 3. Each plug 243 is a structure wide at two ends and narrow in the middle to achieve plug-in fixation.

As shown in Figs. 10 and 11, each expansion mechanism includes a ring-like elastic expansion arm 3, a pair of surfaces of which parallel to the nasal passage axis 97 are its primary surfaces and largest surfaces. The pair of primary surfaces are an inner side primary surface 34 and an outer side primary surface 35. A surface of the elastic expansion arm 3 perpendicular to the nasal passage axis 97 is its secondary surface, the width of which is smaller than the widths of the primary surfaces to reduce respiratory resistance. When worn, the ring-like elastic expansion arm 3 is inserted into the nasal passage 95 and compressed by an inner wall 96 of the nasal passage, and its resilience force causes it to apply an expansion force to the inner wall 96 of the nasal passage, and at the same time, the elastic expansion arm 3 is tightly fit to the inner wall 96 of the nasal passage, and their interaction forces fix the slow release apparatus 1 in the nasal passage 95. The ring-like elastic expansion arm 3 can expand the nasal passage in all directions and is capable of increasing the contact area between the elastic expansion arm 3 and the inner wall 96 of the nasal passage, to reduce the intensity of pressure of the expansion mechanism on the inner wall of the nasal passage, thereby improving comfort.

Fig. 12 depicts a second preferred embodiment in which a forked elastic support spoke 7 is provided. The elastic support spoke 7 includes a first branch 73, a second branch 74 and a third branch 75. The three branches are all elastically compressible arc-shaped strip-like structures, tail ends of which are all connected to an upper surface of the elastic expansion arm 3 and thereby apply to the elastic expansion arm 3 a resilience force generated after the elastic support spoke 7 is compressed. The plurality of branches are conducive to further increasing the strength of expansion as well as the targeting and balance of expansion. Furthermore, the elastic support spoke 7, a fixing tube 5 and a release element 6 are all located above the elastic expansion arm 3, so that the release element 6 can be deeper in the nasal passage 95, which is conducive to releasing a compound to deeper parts of a respiratory tract.

As shown in Figs. 10 and 11, the two release mechanisms are symmetrically arranged, and each release mechanism is correspondingly provided in one of the expansion mechanisms, each release mechanism including a fixing tube 5 and a release element 6.

As shown in Figs. 10 and 11, the fixing tube 5 is a circular tubular structure, which is fixed to the inner side primary surface 34 of the ring-like elastic expansion arm 3, and the fixing tube 5 is used to surround and fix the release element 6. As shown in Fig. 12, as an optimization, the fixing tube 5 is provided on the elastic support spoke 7.

As shown in Figs. 10, 11 and 13, the release element 6 is a structure wide at two ends and narrow in the middle, which includes a tubular medicine barrel 61 and a barrel lid 62 and a barrel bottom 63 connected to and covering two ends of the medicine barrel. Outer diameters of the barrel lid 62 and the barrel bottom 63 are both greater than an outer diameter of the medicine barrel 61. When worn, the release element 6 is fixed to the fixing tube 5, the fixing tube 5 surrounds the medicine barrel 61 and is tightly engaged therewith, and the barrel lid 62 and the barrel bottom 63 are located on outer sides of two ends of the fixing tube 5, with their outer diameters being greater than an inner diameter of the fixing tube 5, so that the release element 6 cannot escape from the fixing tube 5, and at that time, the release element 6 is located in the nasal passage 95. The barrel lid 62 is provided with a columnar protrusion 622, which is provided with a plurality of hemispherical protrusions 624 at an outer side. The columnar protrusion 622 is inserted into an open end of the medicine barrel 61 such that the plurality of hemispherical protrusions 624 are tightly engaged with a plurality of clamping holes 612 formed at the open end of the medicine barrel 61, thereby connecting the medicine barrel 61 and the barrel lid 62. The barrel bottom 63 and the medicine barrel 61 are an integrated structure. A content 64 of the medicine barrel 61 is a volatile solid compound 642. The barrel lid 62 is provided with five first holes 621, and the barrel bottom 63 is provided with a second hole 631, so that a through gas flow is formed in an inner cavity of the medicine barrel 61 to allow the solid compound 642 to volatilize and be released into the nasal passage 95. The release mechanism allows convenient replacement of the content 64 of the medicine barrel, and also allows replacement of the barrel lid 62 with one having a different number of openings, to adjust the release speed, and the release element 6 can also be replaced as a whole with a porous material to accommodate to different release needs.

### Embodiment 3.

Figs. 14, 15, 16, 17A and 17B illustrate a third preferred embodiment of the present invention, in which the slow release apparatus 1 includes: a connection mechanism, two expansion mechanisms and two release mechanisms.

As shown in Figs. 14, 15 and 16, the connection mechanism includes two connecting beams 2, specifically a first connecting beam 21 and a second connecting beam 22 arranged side by side longitudinally. Both the first connecting beam 21 and the second connecting beam 22 semi-surround a lower end of a nasal septum 91. The first connecting beam 21 is a C-shaped structure, which is composed of a first intermediate portion 213 located on a lower side of the nasal septum 91 and two first connecting arms 214 located on two sides of the nasal septum 91. The two first connecting arms 214 are inserted into two nasal passages 95 from the two sides of the nasal septum 91, and are respectively integrally connected to the expansion mechanisms symmetrically arranged in the two nasal passages 95. The second connecting beam 22 is composed of a second intermediate portion 223 located on the lower side of the nasal septum 91 and two second connecting arms 224 located on the two sides of the nasal septum 91. The two second connecting arms 224 are inserted into the two nasal passages 95 from the two sides of the nasal septum 91, and is detachably connected in a plug-in manner to the expansion mechanisms symmetrically arranged in the two nasal passages 95.

As shown in Figs. 14, 15 and 16, since the first connecting beam 21 is an integral structure with the expansion mechanisms, its material cannot provide a sufficient clipping force, and thus the connection mechanism is provided with the second connecting beam 22 to supplement the clipping force. The second connecting beam 22 applies a clipping force to the nasal septum 91 through two parts. A first part is plugs 243 provided at tail ends of the two second connecting arms 224. A clipping force is conducted by the plugs through first elastic support spokes 71 to ring-like elastic expansion arms 3, and then applied to the nasal septum 91 by outer side primary surfaces 35 of the elastic expansion arms 3 close to the nasal septum 91. A second part is two protrusions 242 toward the nasal septum 91 provided at the middle of the two second connecting arms 224. The two protrusions 242 are fit to the two sides of the nasal septum 91 and apply a clipping force to the nasal septum 91. As an optimization, the two first connecting arms 214 of the first connecting beam 21 can be provided with protrusions 242 with the same function.

As shown in Fig. 16, ends of the two second connecting arms 224 of the second connecting beam 22 are provided with plugs 243. The plugs 243 are inserted into jacks 77 provided at the roots of first elastic support spokes 71 to achieve plug-in fixation. Each plug 243 is a structure wide at two ends and narrow in the middle, including a cap 2431, a column 2432 and a shank 2433, wherein maximum widths of the cap 2431 and the shank 2433 are both greater than a maximum width of the column 2432. After installation, the jack 77 surrounds the column 2432 and is tightly engaged therewith, the cap 2431 and shank 2433 are respectively located on outer sides of two ends of the jack 77, with their maximum widths being greater than a maximum width of an inner cavity of the jack 77, so that the plug 243 cannot escape. Preferably, the column 2432 and the inner cavity of the jack 77 are quadrangular-prism structures and are tightly engaged to avoid rotation of the expansion mechanism around the plug 243. As an optimization, ends of the two first connecting arms 214 of the first connecting beam 21 can be provided with same plugs 243, which are connected to the elastic expansion arms 3 or the elastic support spokes 7 in a plug-in manner.

As an optimization, the two first connecting arms 214 of the first connecting beam 21 can be symmetrically provided with widened portions 241, and the width of a surface on a side of each connecting arm 214 facing towards the nasal septum 91 increases at the widened portion 241. The two widened portions 241 are fit to the two sides of the nasal septum 91 and apply a clipping force to the nasal septum 91. As an optimization, the two second connecting arms 224 of the second connecting beam 22 can be symmetrically provided with widened portions 241, and the width of a surface on a side of each second connecting arm 224 facing towards the nasal septum 91 increases at the widened portion 241. The two widened portions 241 are fit to the two sides of the nasal septum 91 and apply a clipping force to the nasal septum 91. The widened portions 241 can be combined with the protrusions 242 into same parts.

As shown in Fig. 14, each expansion mechanism includes an elastic expansion arm 3 and two elastic support spokes 7.

As shown in Figs. 14 and 15, the elastic expansion arm 3 is a ring-like structure, which is connected to an end of the connecting arm 24. A pair of surfaces of the elastic expansion arm 3 parallel to a nasal passage axis 97 are its primary surfaces and largest surfaces. The pair of primary surfaces are an inner side primary surface 34 and an outer side primary surface 35. A surface of the elastic expansion arm 3 perpendicular to the nasal passage axis 97 is its secondary surface, the width of which is smaller than the widths of the primary surfaces to reduce respiratory resistance. The outer side primary surface 35 is fit to an inner wall 96 of the nasal passage, so as to be capable of applying an expansion force from the elastic expansion arm 3 and the elastic support spokes 7 to the nasal passage 95 and applying a clipping force from the second connecting beam 22 to the nasal septum 91. When worn, the elastic expansion arm 3 is compressed by the inner wall 96 of the nasal passage, and its resilience force causes it to apply an expansion force in all directions to the nasal passage 95. At the same time, the elastic expansion arm 3 is tightly fit to the inner wall 96 of the nasal passage, and their interaction forces fix the slow release apparatus 1 in the nasal passage 95. The ring-like elastic expansion arm 3 can expand the nasal passage in all directions and is capable of increasing their contact area, to reduce the intensity of pressure of the expansion mechanism on the inner wall of the nasal passage, thereby improving comfort.

As shown in Figs. 14 and 15, the two elastic support spokes 7 are a first elastic support spoke 71 and a second elastic support spoke 72, which are both band-like structures and are placed spaced apart in the elastic expansion arm 3, i.e., one ends of the first elastic support spoke 71 and the second elastic support spoke 72 are connected to the inner side primary surface 34 of the elastic expansion arm 3 close to the nasal septum 91, and the other ends thereof are connected to the inner side primary surface 34 of the elastic expansion arm 3 close to the nasal wing 92, thereby ensuring that their resilience force is applied to the nasal wing 92 in a targeted manner.

As shown in Figs. 14 and 15, the two release mechanisms are symmetrically arranged, and each release mechanism is correspondingly provided in one of the expansion mechanisms, each release mechanism including a fixing tube 5 and a release element 6.

As shown in Fig. 14, the fixing tube 5 has an inner cavity of a quadrangular-prism structure, and is fixed to the second elastic support spoke 72, and the fixing tube 5 surrounds and fixes the release element 6. As an optimization, the fixing tube 5 can also be provided on the support spoke 71, or on the inner side primary surface 34 of the elastic expansion arm 3.

As shown in Figs. 14 and 17A, the release element 6 is a structure wide at two ends and narrow in the middle, and its two ends and middle are quadrangular-prism structures. When worn, the release element 6 is fixed to the fixing tube 5, the fixing tube 5 surrounds the middle of release element 6 and is tightly engaged therewith, the quadrangular-prism structures of the middle of the release element 6 and the inner cavity of the fixing tube can avoid rotation of release element 6, and the two ends of the release element 6 are located on outer sides of two ends of the fixing tube 5, with their maximum widths being greater than a maximum width of the inner cavity of the fixing tube 5, so that the release element 6 cannot escape from the fixing tube 5. When worn, the release element 6 is located in the nasal passage 95.

The release element 6 is made of a porous material capable of absorbing and holding liquid compounds. Its two wide ends and porous surface are conducive to compound volatilization, and also conducive to an inspiratory airflow in the nasal passage carrying away tiny compound droplets. In particular, the porous material is capable of releasing non-volatile liquid compounds in the form of tiny droplets.

As shown in Fig. 17B, as an optimization, two ends of the release element 6 made of the porous material are provided with bulges 65 to further increase its surface area and increase the compound release speed.

Finally, it should be noted that the above embodiments are only used for describing instead of limiting the technical solutions of the present invention. Although the present invention is described in detail with reference to the embodiments, persons of ordinary skill in the art should understand that modifications or equivalent substitutions of the technical solutions of the present invention should be encompassed within the scope of the claims of the present invention so long as they do not depart from the spirit and scope of the technical solutions of the present invention.

## Claims

1. A slow release apparatus for slowly releasing a compound in a nasal passage, wherein the slow release apparatus (1) comprises a connection mechanism, two expansion mechanisms, and two release mechanisms; two ends of the connection mechanism are respectively integrally or detachably connected to the two expansion mechanisms symmetrically arranged in nasal passages (95) at two sides; and the two release mechanisms are symmetrically arranged, and each release mechanism is correspondingly provided in one of the expansion mechanisms;
the connection mechanism comprises at least one connecting beam (2), the connecting beam (2) semi-surrounding a lower end of a nasal septum (91) and comprising an intermediate portion (23) located on a lower side of the nasal septum (91) and two connecting arms (24) symmetrically arranged on two sides of the nasal septum (91); the intermediate portion (23) is located between the two connecting arms (24); the two connecting arms (24) are respectively connected to the two expansion mechanisms symmetrically arranged in the two nasal passages (95);
the release mechanism comprises at least one fixing tube (5) and at least one release element (6);
the fixing tube (5) is a tubular structure, which is arranged on the expansion mechanism; the release element (6) is a structure wide at two ends and narrow in the middle, and when worn, the release element (6) is fixed in the fixing tube (5), the fixing tube (5) surrounds the thinner middle of the release element (6) and is tightly engaged therewith, the two ends of the release element (6) are located on outer sides of two ends of the fixing tube (5), and maximum widths of the two ends of the release element (6) are greater than a maximum width of an inner cavity of the fixing tube (5); and the release element (6) is capable of containing a certain amount of compound and allows the contained compound to be slowly released in the nasal passage (95), and when worn, at least part of the release element (6) is located in the nasal passage (95).

2. The slow release apparatus for slowly releasing a compound in a nasal passage according to claim 1, wherein the expansion mechanism comprises an elastic expansion arm (3); the elastic expansion arm (3) is a ring-like closed structure, which is connected to an end of one connecting arm (24); the fixing tube (5) is arranged on an inner side primary surface (34) of the elastic expansion arm (3), and the release element (6) is sleeved in the fixing tube (5).

3. The slow release apparatus for slowly releasing a compound in a nasal passage according to claim 2, wherein the two connecting arms (24) of the connecting beam (2) are symmetrically provided with widened portions (241), and the width of a surface on a side of each connecting arm (24) facing towards the nasal septum (91) increases at the widened portion (241); and the two widened portions (241) are fit to two sides of the nasal septum (91) and apply a wrap-around force to the nasal septum (91).

4. The slow release apparatus for slowly releasing a compound in a nasal passage according to claim 1, wherein the expansion mechanism comprises an elastic expansion arm (3) and at least one elastic support spoke (7);
the elastic expansion arm (3) is a ring-like closed structure, which is connected to an end of one connecting arm (24); the elastic support spoke (7) is forked and comprises a plurality of branches, each branch having one end connected to a trunk part of the elastic support spoke (7), and the other end fixed to the elastic expansion arm (3); and the fixing tube (5) is arranged on the elastic support spoke (7), and the release element (6) is sleeved in the fixing tube (5).

5. The slow release apparatus for slowly releasing a compound in a nasal passage according to claim 4, wherein the elastic support spoke (7), the fixing tube (5) and the release element (6) are located above the elastic expansion arm (3), forming an inverted bowl structure.

6. The slow release apparatus for slowly releasing a compound in a nasal passage according to claim 1, wherein the expansion mechanism comprises an elastic expansion arm (3) and at least one elastic support spoke (7);
the elastic expansion arm (3) is a ring-like closed structure, which is connected to an end of one connecting arm (24); and the elastic support spoke (7) is a band-like structure, two ends of which are connected to the elastic expansion arm (3).

7. The slow release apparatus for slowly releasing a compound in a nasal passage according to claim 6, wherein the connection mechanism comprises a first connecting beam (21) and a second connecting beam (22) arranged side by side longitudinally; and both the first connecting beam (21) and the second connecting beam (22) semi-surround the lower end of the nasal septum (91).

8. The slow release apparatus for slowly releasing a compound in a nasal passage according to claim 7, wherein two second connecting arms (224) of the second connecting beam (22) are symmetrically provided with protrusions (242) towards the nasal septum (91) of a wearer; and the protrusions (242) are fit to the two sides of the nasal septum (91) and apply a wrap-around force to the nasal septum (91).

9. The slow release apparatus for slowly releasing a compound in a nasal passage according to claim 7, wherein ends of two second connecting arms (224) of the second connecting beam (22) are provided with plugs (243), the plugs (243) being inserted into jacks (77) provided at roots of first elastic support spokes (71), each plug (243) being a structure wide at two ends and narrow in the middle to achieve plug-in fixation.

10. The slow release apparatus for slowly releasing a compound in a nasal passage according to claim 1, wherein the expansion mechanism comprises an elastic expansion arm (3), a nasal passage engagement element (4) and at least one elastic support spoke (7);
the elastic expansion arm (3) is an elastically compressible open band-like structure, which is connected to an end of one connecting arm (24), and an outer side primary surface (35) of the elastic expansion arm (3) is configured to be partially or entirely fit to an inner wall (96) of the nasal passage, so as to be capable of applying an expansion force from the elastic expansion arm (3) and the elastic support spoke (7) to a nasal wing (92) and applying a wrap-around force from the connecting beam (2) to the nasal septum (91);
the nasal passage engagement element (4) is a sheet-like structure, which is connected to the elastic expansion arm (3) and keeps the expansion mechanism as an open structure; when worn, the nasal passage engagement element (4) is located on an inner side of the nasal wing (92), and an outer primary surface (42) thereof is fit to an inner wall of the nasal wing (92) and applies an expansion force from the elastic expansion arm (3) and the elastic support spoke (7) to the nasal wing (92); and
the elastic support spoke (7) is an elastically compressible band-like or strip-like structure, two ends of which are connected to the elastic expansion arm (3) or the nasal passage engagement element (4).

11. The slow release apparatus for slowly releasing a compound in a nasal passage according to claim 10, wherein the elastic expansion arm (3) is fused with the nasal passage engagement element (4) into an integral whole.

12. The slow release apparatus for slowly releasing a compound in a nasal passage according to claim 10, wherein a transverse cross-section of the elastic expansion arm (3) perpendicular to a nasal passage axis (97) is a V-shaped structure or C-shaped structure comprising a bent portion (31), a proximal arm (32) and a distal arm (33);
the bent portion (31) is connected to the proximal arm (32) at one end and to the distal arm (33) at the other end; and when worn, the proximal arm (32) is close to the nasal septum (91), the distal arm (33) is close to the nasal wing (92), and the bent portion (31) is close to a nasal bridge (93) or a nasal base (99).

13. The slow release apparatus for slowly releasing a compound in a nasal passage according to claim 10, wherein an end of the elastic expansion arm (3), at a part fit to the inner wall (96) of the nasal passage, is bent towards an inner side of the nasal passage (95); and an end of the nasal passage engagement element (4) is bent towards the inner side of the nasal passage (95).

14. The slow release apparatus for slowly releasing a compound in a nasal passage according to claim 10, wherein the elastic support spoke (7) is forked and has a plurality of branches, each branch having one end connected to a trunk part of the elastic support spoke (7), and the other end fixed to the elastic expansion arm (3) or the nasal passage engagement element (4).

15. The slow release apparatus for slowly releasing a compound in a nasal passage according to claim 1, wherein the connecting beam (2) applies a wrap-around force to the nasal septum (91), the wrap-around force being applied directly to the lower end of the nasal septum (91) by the two connecting arms (24) of the connecting beam (2), or after being conducted by the two connecting arms (24) of the connecting beam (2) to the corresponding expansion mechanisms, the wrap-around force being applied to the lower end of the nasal septum (91) by the corresponding expansion mechanisms.

16. The slow release apparatus for slowly releasing a compound in a nasal passage according to claim 1, wherein an average transverse cross-sectional area of the intermediate portion (23) of the connecting beam (2) is larger than that of the connecting arms (24).

17. The slow release apparatus for slowly releasing a compound in a nasal passage according to claim 1, wherein the release element (6) comprises a medicine barrel (61), and a barrel lid (62) and a barrel bottom (63) connected to and covering two ends of the medicine barrel (61); a maximum diameter of the barrel lid (62) is greater than a maximum diameter of the medicine barrel (61); a maximum diameter of the barrel bottom (63) is greater than the maximum diameter of the medicine barrel (61); and
the medicine barrel (61) is a hollow tubular structure, which contains a compound therein, and the barrel lid (62) and the barrel bottom (63) are provided with at least one opening to allow the compound to be discharged through the opening towards the nasal passage (95).

18. The slow release apparatus for slowly releasing a compound in a nasal passage according to claim 17, wherein the medicine slow-release barrel (61) is provided with an absorbent block (641) made of a porous material therein.

19. The slow release apparatus for slowly releasing a compound in a nasal passage according to claim 1, wherein the release element (6) is made of a porous material.

20. The slow release apparatus for slowly releasing a compound in a nasal passage according to claim 19, wherein an outer surface of the release element (6) is provided with bulges (65).
